# EUROPEAN PATENT APPLICATION

(11) **EP 1 441 033 A2**
(43) Date of publication of application: **28.07.2004**
(21) Application number: 04008341.2
(22) Date of filing: 12.01.1998
(51) Int. Cl.: C12N 15/86, A61K 35/76, A61K 39/00

(54) **Eukaryotic gene expression cassette based on Herpes simplex virus**

(30) Priority: 10.01.1997 GB 9700411
(62) Divisional of application: 98900570.7
(71) Applicant: BioVex Limited, Oxford OX14 4RX (GB)
(72) Inventor: Coffin, Robert Stuart, Abingdon Oxford OX14 4RX (GB); Latchman, David Seymour, Bloomsbury WC1E 7HX (GB)
(74) Representative: Woods, Geoffrey Corlett

(57) **Abstract**

An expression cassette comprising a herpes simplex virus latency-associated transcript P2 region, a promoter and a heterologous gene operably linked in that order. The expression cassette is incorporated into herpes simplex virus vectors to allow for delivery of heterologous genes to mammalian cells for long-term expression.

## Description

### Field of the Invention

The present invention relates to a gene expression cassette. The expression cassette can be used for directing long-term expression of heterologous genes in eukaryotic cells. It also relates to the use of said expression cassette in gene therapy, vaccine production, and in methods of assaying for gene function. It further relates to vectors, including viral strains, comprising said expression cassette.

### Background to the invention

Herpes simplex virus (HSV) has often been suggested as a suitable gene-delivery vector for the nervous system due to its neurotrophic lifestyle and its ability to remain latent in neurons for the lifetime of the cell. This unique ability has suggested that with suitable development a once-only application of such a vector system might give a lifelong therapeutic benefit for certain conditions, such as Parkinson's disease where expression of tyrosine hydroxylase or GDNF in the brain has been shown to be beneficial.

However, while disabled herpes viruses have been shown efficiently to deliver genes to the nervous system and to other tissues in *vivo,* transcription of heterologous genes expressed from the herpes genome invariably only continues in the short term (< 1 week). Transcription is shutoff as the herpes genome takes up the transcriptionally inactive state maintained during virus latency. Thus while herpes vector DNA probably remains in the treated cell for the lifetime of that cell, a therapeutic benefit would only be shown in the short term as the heterologous gene, while present, is usually not transcribed.

It follows that if promoter systems could be developed which remain active during latency the full potential of herpes virus vectors would be realised as a therapeutic benefit would continue during viral latency. A further desirable property of such promoters would be that they only gave activity in the cell types to be treated, i.e. neurons or subsets of neurons or other specific cell types, thus preventing the inappropriate expression of a potentially therapeutic gene which might in some cases be harmful. The 'gene-therapy' would thus be confined to the target cells to be treated.

Herpes simplex viruses, causing cold-sores (HSV1) or genital herpes (HSV2), infect the axonal terminals of sensory neurons through abrasions on the skin or mucosal surface, following which they migrate to the nucleus in the cell-body where either a latent infection is established or lytic replication occurs. The factors influencing the decision for a latent or lytic infection are not well understood. However, in the case of a disabled virus vector only the latent pathway is possible.

During latency the herpes genome is largely transcriptionally inactive. However a small region within the long repeats of the genome is transcribed producing the latency associated transcripts (LATs), here described for HSV1 although HSV2 is similar. The LATs fall into two classes which are co-linear, a large approximately 8 kilobase (kb) transcript of very low abundance transcribed from a TATA-containing promoter (see Coffin and Latchman, 1996; here termed LAT P1), and smaller highly abundant transcripts of approximately 2 kb and 1.5 kb which are thought to be nested stable introns spliced from the larger transcript. The 1.5 kb transcript is only detected in neurons and the abundance of both increases during latency. A second region with very weak promoter activity (see Goins *et al.,* 1994; here termed LAT P2) has been identified between LAT P1 and the start of the 2 kb LAT suggesting to some that it might be expressed as a separate transcript under some circumstances.

The use of LAT promoter regions for driving the long-term expression of heterologous genes inserted into the viral genome has met with little success. Use of the LAT P1 promoter leads to high expression levels in the short term but transcription is rapidly shut off after a few days. Insertion of heterologous genes at various positions after the endogenous LAT P2 promoter does give long-term activity, but this activity is very weak (see Coffin and Latchman, 1996). Similarly, a construct inserted outside the LAT region (in the glycoprotein C region) consisting of a LAT P2 promoter linked to a *lacZ* coding region has been reported to give long-term activity in dorsal root ganglia *in vitro,* but this activity was also very weak (e.g. Goins *et al.,* 1994).

### Summary of the Invention

The present invention relates to an expression cassette comprising a herpes simplex virus LAT P2 region, a promoter and a heterologous gene operably linked in that order. The present invention is based on the surprising finding that the LAT P2 region can confer long-term activity on an adjacent promoter. Importantly, the use of the LAT P2 region and an adjacent promoter to drive expression of a heterologous gene results in not only long-term expression of the heterologous gene, but also in high levels of expression. A single LAT P2 element may also be used to drive long term expression from pairs of promoters. Here a centrally located LAT P2 element is flanked by two promoters facing away from it in opposite orientations, such that two heterologous genes can be operably linked to these promoters resulting in the long term expression of both genes.

Our hypothesis is that the region of the HSV genome previously referred to as the LAT P2 (Coffin and Latchman, 1996) or LAP 2 promoter (Goins *et al.,* 1994) does not itself provide promoter activity during latency. Instead, it provides an altered DNA structure in surrounding regions allowing continued transcription from nearby promoters in an otherwise transcriptionally inactive latent genome.

Nucleic acid constructs, including virus strains, in particular HSV strains, comprising said expression cassette can be used, for example, for delivering therapeutic genes in methods of treatment of diseases of, or injuries to, for example, the nervous system, including Parkinson's disease, spinal injury or strokes, or diseases of the eye, heart or skeletal muscles, or malignancies, or for the delivery of genes encoding specific antigens for vaccine purposes.

The present invention also relates to methods for studying the function of genes in eukaryotic cells, especially mammalian cells, for example in identifying genes complementing cellular dysfunctions, or studying the effect of expressing mutant genes in wild-type or mutant cells. The methods of the present invention may be used in particular for the functional study of genes implicated in disease.

Accordingly the present invention provides an expression cassette comprising a HSV latency-associated transcript P2 region, a promoter and a heterologous gene operably linked in that order. Preferably the promoter is a non-latency-associated transcript promoter. More preferably the promoter is a viral promoter or mammalian promoter. Preferably the promoter is a viral promoter or mammalian promoter permitting expression of the heterologous gene in a mammalian cell, preferably a cell of the central or peripheral nervous system, or a cell of the eye, heart or skeletal muscle, more preferably a cell of the central or peripheral nervous system. The promoter may also be inducible and/or tissue-specific.

The expression cassette, or vector, preferably a viral strain, more preferably an HSV strain, comprising the expression cassette of the invention may thus be used to deliver a heterologous gene to a mammalian cell where it will be expressed. Such expression cassettes and vectors are useful in a variety of applications, for example, in gene therapy, as vaccines, in *in vitro* or *in vivo* assay methods or for the study of gene function.

The term heterologous gene is intended to embrace any gene not found in the HSV genome. The heterologous gene may be any allelic variant of a wild-type gene, or it may be a mutant gene. The heterologous gene preferably encodes a polypeptide of therapeutic use, including polypeptides which are cytotoxic or capable of converting a precursor prodrug into a cytotoxic compound.

Gene therapy and other therapeutic applications may well require the administration of multiple genes. The expression of multiple genes may be advantageous for the treatment of a variety of conditions - e.g. using multiple neurotrophic factors. HSV is uniquely appropriate as it does not have the limited packaging capabilities of other viral vector systems. Thus multiple heterologous genes can be accommodated within its genome. There are, for example, at least two ways in which this could be achieved. For example, more than one expression cassette of the invention could be introduced into a particular HSV strain. Each expression cassette may comprise one heterologous gene. An alternative approach would be to use pairs of promoters (the same or different promoters) facing in opposite orientations away from a centrally located LAT P2 element, these promoters each driving the expression of a heterologous gene (the same or different heterologous gene).

Thus the present invention also provides an expression cassette further comprising a second promoter and a second heterologous gene operably linked in that order to said HSV LAT P2 region and in the opposite orientation to the first promoter and first heterologous gene. The second promoter may be the same as or different to the first promoter. The second heterologous gene may be the same as or different to the first heterologous gene. In a particularly preferred embodiment, the first promoter is a non-LAT promoter and the second promoter is a LAT P1 promoter.

In summary this arrangement provides a pair of promoter/heterologous gene constructs in opposite orientations flank a single LAT P2 region, allowing the long-term expression of pairs of genes, which may be the same or different, driven by the same or different promoters.

A combinatorial approach could also be used where one or more of the first type of expression cassette is introduced into the genome of the HSV strain together with one or more of the second type of expression cassette. Consequently, where appropriate, references to "the expression cassette" should be taken to include multiple expression cassettes of either type.

The invention further provides for the use of the expression cassette and vectors, preferably HSV strains, comprising the expression cassette, for use in the treatment of humans and animals. For example, HSV strains comprising the expression cassette can be used in the treatment of a disease of, or injury to, the nervous system, including Parkinson's disease, spinal injury or stroke or a disease of the eye, heart or skeletal muscle, or a malignancy. Such HSV strains will have been attenuated so that they are unable to undergo the lytic cycle in human and animal cells.

The expression cassette of the present invention may also be used in methods for studying the function of genes in eukaryotic cells, preferably mammalian cells, for example in identifying genes complementing cellular dysfunctions, or studying the effect of expressing mutant genes in wild-type or mutant mammalian cells. The methods of the present invention may be used in particular for the functional study of genes implicated in disease.

The invention also provides a method for producing a viral strain, preferably an HSV strain, comprising an expression cassette of the invention, which method comprises introducing an expression cassette of the invention into the genome of the virus strain, preferably by homologous recombination.

### Detailed Description of the Invention

### A. Expression Cassette - LAT P2 region, promoter(s), heterologous gene(s)

The expression cassette of the invention consists essentially of a LAT P2 region, a promoter and a heterologous gene operably linked in that order. The term "operably linked" refers to a juxtaposition wherein the components are in a relationship permitting them to function in their intended manner. Thus, for example, a promoter operably linked to a heterologous gene sequence is ligated in such a way that expression of the heterologous gene is achieved under conditions which are compatible with the activation of expression from the promoter.

The expression cassette may further comprising a second promoter and a second heterologous gene operably linked in that order to said HSV LAT P2 region and in the opposite orientation to the first promoter and first heterologous gene wherein said second promoter and second heterologous gene are the same as or different to the first promoter and first heterologous gene. Thus a pair of promoter/heterologous gene constructs in opposite orientations flank a single LAT P2 region allowing the long-term expression of pairs of heterologous genes, which may be the same or different, driven by the same or different promoters. Furthermore, the product of the first heterologous gene may regulate the expression of the second heterologous gene (or vice-versa) under suitable physiological conditions.

The term "long-term expression" is taken to mean expression of a heterologous gene in a cell infected with a herpes simplex virus of the invention even after the herpes simplex virus has entered latency. Preferably, this is for at least two weeks, more preferably at least one or two months after infection, even more preferably for the life-time of the cell.

The expression cassette can be constructed using routine cloning techniques known to persons skilled in the art (see, for example, Sambrook *et al.,* 1989, Molecular Cloning - a laboratory manual; Cold Spring Harbor Press).

### 1. LAT P2 region

The LAT P2 region is here defined as HSV1 nucleotides 118,866-120,219 (GenBank HE1CG: from *Pst*I*-BstX*I sites), fragments or derivatives of this region, including homologous regions of HSV2, which are capable of providing for long-term expression of heterologous genes in the expression cassette of this invention.

### 2. Promoter

A promoter means a transcriptional promoter not derived from the LAT P2 region of HSV. The term promoter is well-known in the art and encompasses nucleic acid regions ranging in size and complexity from minimal promoters to promoters including upstream elements and enhancers. The promoter is operably linked to and downstream of the LAT P2 region. It is also possible to operably link in the reverse orientation an additional promoter upstream of the LAT P2 region such that the LAT P2 region confers long-term activity on both promoters.

The promoter is selected from promoters which are functional in mammalian, preferably human, cells. The promoter may be derived from promoter sequences of viral or eukaryotic genes. For example, it may be a promoter derived from the genome of a cell in which expression of the heterologous gene is to occur, preferably a cell of the mammalian central or peripheral nervous system. With respect to eukaryotic promoters, they may be promoters that function in a ubiquitous manner (such as promoters of α-actin,β-actin, tubulin) or, alternatively, a tissue-specific manner (such as promoters of the genes for pyruvate kinase). Promoters which are active in only certain neuronal cell types are especially preferred (for example the tyrosine hydroxylase (TH), L7, or neuron specific enolase (NSE) promoters). They may also be promoters that respond to specific stimuli, for example promoters that bind steroid hormone receptors. Viral promoters may also be used, for example the Moloney murine leukaemia virus long terminal repeat (MMLV LTR) promoter, the HSV1 or HSV2 LAT P1 promoter, the rous sarcoma virus (RSV) LTR promoter or the human cytomegalovirus (CMV) IE promoter.

It may also be advantageous for the promoters to be inducible so that the levels of expression of the heterologous gene can be regulated during the life-time of the cell. Inducible means that the levels of expression obtained using the promoter can be regulated. For example, in a preferred embodiment where two promoters in opposite orientations flank a single LAT P2 region one promoter would comprise a promoter responsive to the tet repressor/VP16 transcriptional activator fusion protein previously reported (Gossen and Bujard, 1992; Gossen *et al,* 1995), and driving the heterologous gene the expression of which is to be regulated. The second promoter would comprise a strong promoter (e.g. the CMV IE promoter) driving the expression of the tet repressor/VP16 fusion protein. Thus in this example expression of the first heterologous gene would depend on the presence or absence of tetracycline.

In addition, any of these promoters may be modified by the addition of further regulatory sequences, for example enhancer sequences. Chimeric promoters may also be used comprising sequence elements from two or more different promoters described above.

### 3. Heterologous genes

The term "nucleic acid" includes ribonucleic acid, deoxyribonucleic acid and analogues thereof. The term heterologous gene comprises any gene other than one present in the HSV genome. The heterologous gene may be any allelic variant of a wild-type gene, or it may be a mutant gene. The term "gene" is intended to cover nucleic acid sequences which are capable of being at least transcribed. Thus, sequences encoding mRNA, tRNA and rRNA are included within this definition. The sequences may be in the sense or antisense orientation with respect to the promoter. Antisense constructs can be used to inhibit the expression of a gene in a cell according to well-known techniques. Sequences encoding mRNA will optionally include some or all of 5' and/or 3' transcribed but untranslated flanking sequences naturally, or otherwise, associated with the translated coding sequence. It may optionally further include the associated transcriptional control sequences normally associated with the transcribed sequences, for example transcriptional stop signals, polyadenylation sites and downstream enhancer elements.

The heterologous gene may encode, for example, proteins involved in the regulation of cell division, for example growth factors including neurotrophic growth factors (such as brain-derived neurotrophic factor, glial cell derived neurotrophic factor, NGF, NT3, NT4 and NT5), cytokines (such as α-, β- or γ-interferon, interleukins including IL-1, IL-2, tumour necrosis factor, or insulin-like growth factors I or II), protein kinases (such as MAP kinase), protein phosphatases and cellular receptors for any of the above. The heterologous gene may also encode enzymes involved in cellular metabolic pathways, for example enzymes involved in amino acid biosynthesis or degradation (such as tyrosine hydroxylase), purine or pyrimidine biosynthesis or degradation, and the biosynthesis or degradation of neurotransmitters, such as dopamine, or protein involved in the regulation of such pathways, for example protein kinases and phosphatases. The heterologous gene may also encode transcription factors or proteins involved in their regulation, for example members of the Brn3 family or pocket proteins of the Rb family such as Rb or p107, membrane proteins (such as rhodopsin), structural protein (such as dystrophin) or heat shock proteins such as hsp70.

Preferably, the heterologous gene encodes a polypeptide of therapeutic use, or the function of which may be implicated in a disease process. For example, of the proteins described above, tyrosine hydroxylase and glial cell derived neurotrophic factor can be used in the treatment of Parkinson's disease, rhodopsin can be used in the treatment of eye disorders, dystrophin may be used to treat muscular dystrophy, and heat shock proteins can be used to treat disorders of the heart and brain associated with ischaemic stress. Polypeptides of therapeutic use may also include cytotoxic polypeptides such as ricin, or enzymes capable of converting a precursor prodrug into a cytotoxic compound for use in, for example, methods of virus-directed enzyme prodrug therapy or gene-directed enzyme prodrug therapy. In the latter case, it may be desirable to ensure that the enzyme has a suitable signal sequence for directing it to the cell surface, preferably a signal sequence that allows the enzyme to be exposed on the exterior of the cell surface whilst remaining anchored to cell membrane. Suitable enzymes include bacterial nitroreductase such as *E. coli* nitroreductase as disclosed in WO93/08288 or carboxypeptidase, especially carboxypeptidase CPG2 as disclosed in W088/07378. Other enzymes may be found by reference to EP-A-415731. Suitable prodrugs include nitrogen mustard prodrugs and other compounds such as those described in WO88/07378, WO89/10140, WO90/02729 and WO93/08288 which are incorporated herein by reference.

Heterologous genes may also encode antigenic polypeptides for use as vaccines. Preferably such antigenic polypeptides are derived from pathogenic organisms, for example bacteria or viruses, or from tumours.

Heterologous genes may also include marker genes (for example encoding β-galactosidase or green fluorescent protein) or genes whose products regulate the expression of other genes (for example, transcriptional regulatory factors including the tet repressor/VP16 transcriptional activator fusion protein described above).

### B. Vectors

The expression cassette may be used in the form of a naked nucleic acid construct. Alternatively, it may be introduced into a variety of nucleic acid vectors. Such vectors include plasmids and viral vectors, preferably HSV vectors. Vectors may further include sequences flanking the expression cassette which comprise sequences homologous to eukaryotic genomic sequences, preferably mammalian genomic sequences, or viral genomic sequences. This will allow the introduction of the expression cassette into the genome of eukaryotic cells or viruses by homologous recombination. In particular, a plasmid vector comprising the expression cassette flanked by viral sequences, preferably HSV1 or HSV2 sequences, can be used to prepare a viral vector, preferably an HSV vector, suitable for delivering the expression cassette to a mammalian cell. This is described in further detail below for the herpes simplex virus. However the techniques employed are well-known to a skilled person and will be suitable for other viruses such as adenoviruses. Other examples of suitable viral vectors include viral vectors able to integrate their genomes into the host cell genome, for example retroviruses, including lentiviruses, and adeno-associated virus.

### C. Herpes Simplex Virus Vectors

### 1. Viral Strains

The HSV strains of the invention comprising the expression cassette may be derived from, for example, HSV1 or HSV2 strains, or derivatives thereof, preferably HSV1. Derivatives include inter-type recombinants containing DNA from HSV1 and HSV2 strains. Derivatives preferably have at least 70% sequence homology to either the HSV1 or HSV2 genomes, more preferably at least 90%, even more preferably 95%.

The use of HSV strains in therapeutic procedures will require the strains to be attenuated so that they cannot establish a lytic cycle. In particular, if HSV vectors are to be used for gene therapy in humans the expression cassette should preferably be inserted into an essential gene. This is because if a vector virus encounters a wild-type virus transfer of a heterologous gene to the wild-type virus could occur by recombination. However as long as the heterologous is inserted into an essential gene this recombinational transfer would also delete the essential gene in the recipient virus and prevent 'escape' of the heterologous gene into the replication competent wild-type virus population.

Attenuated strains may be used to produce the HSV strain of the present invention, here given as examples only, including strains that have mutations in either ICP34.5 or ICP27, for example strain 1716 (MacLean *et al.,* 1991), strains R3616 and R4009 (Chou and Roizman, 1992) and R930 (Chou *et al.,* 1994) all of which have mutations in ICP34.5, and d27-1 (Rice and Knipe, 1990) which has a deletion in ICP27. Alternatively strains deleted for ICP4, ICP0, ICP22, ICP6, ICP47, *vhs* or gH, with an inactivating mutation in VMW65, or with any combination of the above may also be used to produce HSV strains of the invention.

The terminology used in describing the various HSV genes is as found in Coffin and Latchman, 1996.

### 2. Complementing cell lines

HSV viruses defective in ICP27 are propagated in a cell line expressing ICP27, for example V27 cells (Rice and Knipe, 1990), 2-2 cells (Smith *et al.,* 1992) or B130/2 cells (see the Examples), preferably B130/2 cells.

ICP27-expressing cell lines can be produced by co-transfecting mammalian cells, for example the Vero or BHK cells, with a vector, preferably a plasmid vector, comprising a functional HSV ICP27 gene capable of being expressed in said cells, and a vector, preferably a plasmid vector, encoding a selectable marker, for example neomycin resistance. Clones possessing the selectable marker are then screened further to determine which clones also express functional ICP27, for example on the basis of their ability to support the growth of ICP27⁻ mutant HSV strains, using methods known to those skilled in the art (for example as described in Rice and Knipe, 1990).

Cell lines which do not allow reversion of an ICP27⁻ mutant HSV strain to a strain with functional ICP27 are produced as described above, ensuring that the vector comprising a functional ICP27 gene does not contain sequences that overlap with (i.e. are homologous to) sequences remaining in the ICP27⁻ mutant virus.

Where HSV strains of the invention comprise inactivating modifications in other essential genes, for example ICP4, complementing cell lines will further comprise a functional HSV gene which complements the modified essential gene in the same manner as described for ICP27.

### 3. Methods of mutation

HSV genes may be rendered functionally inactive by several techniques well known in the art. For example, they may be rendered functionally inactive by deletions, substitutions or insertions, preferably by deletion. Deletions may remove portions of the genes or the entire gene. Inserted sequences may include the expression cassette described above.

Mutations are made in the HSV strains by homologous recombination methods well-known to those skilled in the art. For example, HSV genomic DNA is transfected together with a vector, preferably a plasmid vector, comprising the mutated sequence flanked by homologous HSV sequences. The mutated sequence may comprise deletions, insertions or substitutions, all of which may be constructed by routine techniques. Insertions may include selectable marker genes, for example *lacZ,* for screening recombinant viruses by, for example, β-galactosidase activity.

Mutations may also be made in other HSV genes, for example genes such as ICP0, ICP4, ICP6, ICP22, ICP47, VMW65, gH or *vhs.* In the case of the VMW65 gene, the entire gene is not deleted since it encodes an essential structural protein, but a small inactivating insertion is made which abolishes the ability of VMW65 to transcriptionally activate IE genes (Ace *et al.,* 1989).

### 4. HSV strains comprising the expression cassette

The expression cassette may be inserted into the HSV genome at any location provided that the virus can still be propagated, which may require the use of a cell line carrying another HSV essential gene (as described in 2.) if the heterologous gene is inserted into an essential gene. For example, if the heterologous gene is inserted into the ICP27 gene of the HSV strain, then a cell-line expressing ICP27 would be needed.The expression cassette is preferably inserted into the region of the ICP27 mutation as in the unlikely event that the mutation is repaired by recombination with a wild-type virus, the repair would remove the inserted expressioncassette.

The expression cassette may be inserted into the HSV genome by homologous recombination of HSV strains with, for example, plasmid vectors carrying the expression cassette flanked by HSV sequences, as described above for introducing mutations. The expression cassette may be introduced into a suitable plasmid vector comprising HSV sequences using cloning techniques well-known in the art.

It is possible to insert more than one expression cassette into the viral genome, thus a viral vector may comprise more than one expression cassette of the invention.

It is also possible to make use of the endogenous LAT P2 region by cloning constructs, comprising a promoter operably linked to a heterologous gene, downstream of the endogenous LAT P2 region. Another construct may be cloned upstream of the LAT P2 region but in the opposite orientation. The resulting virus will thus comprise an expression cassette of the invention but produced by slightly different means to those described above.

### D. Administration

The expression cassette of the invention may thus be used to deliver therapeutic genes to a human or animal in need of treatment. In particular, the neurotrophic nature of the herpes simplex virus makes the use of attenuated HSV strains comprising the expression cassette of the invention ideally suited for the treatment of, for example, Parkinson's disease, disorders of the nervous system, spinal injury, strokes or malignancies, for example gliomas. Alternatively, the expression cassette of the invention may be used to deliver genes encoding potentially immunogenic polypeptides for vaccine purposes.

The expression cassette of the invention may be administered directly as a naked nucleic acid construct, preferably further comprising flanking sequences homologous to the host cell genome. Uptake of naked nucleic acid constructs by mammalian cells is enhanced by several known techniques including biolistic transformation and lipofection.

Alternatively, the expression cassette may be administered as part of a nucleic acid vector, including a plasmid vector or viral vector, preferably HSV.

Preferably the delivery vehicle (i.e. naked nucleic acid construct or viral vector comprising the expression cassette for example) is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, intracranial, subcutaneous, intraocular or transdermal administration.

The pharmaceutical composition is administered in such a way that the expression cassette containing the therapeutic gene for gene therapy, can be incorporated into cells at an appropriate area. For example, when the target of gene therapy is the central or peripheral nervous system and the expression cassette is to be delivered by a herpes simplex virus vector, the composition could be administered in an area where synaptic terminals are located so that the virus can be taken up into the terminals and transported in a retrograde manner up the axon into the axonal cell bodies via retrograde axonal transport. The pharmaceutical composition is typically administered to the brain by stereotaxic inoculation. When the pharmaceutical composition is administered to the eye, sub-retinal injection is typically the technique used.

When the expression cassette is delivered to cells by a viral vector, the amount of virus administered is in the range of from 10³ to 10¹⁰ pfu, preferably from 10⁵ to 10⁸ pfu, more preferably from 10⁶ to 10⁷ pfu. When injected, typically 1-10 µl of virus in a pharmaceutically acceptable suitable carrier or diluent is administered.

When the expression cassette is administered as a naked nucleic acid, the amount of nucleic acid administered is typically in the range of from 1 µg to 10 mg, preferably from 100 µg to 1 mg.

Where the heterologous gene is under the control of an inducible promoter, it may only be necessary to induce gene expression for the duration of the treatment. Once the condition has been treated, the inducer is removed and expression of the heterologous gene is stopped. This will clearly have clinical advantages. Such a system may, for example, involve administering the antibiotic tetracycline, as described above, to activate gene expression via its effect on the tet repressor/VP16 fusion protein.

The use of tissue-specific promoters will be of assistance in the treatment of disease using the expression cassette of the invention. For example, several neurological disorders are due to aberrant expression of particular gene products in only a small subset of cells. It will be advantageous to be able express therapeutic genes in only the relevant affected cell types, especially where such genes are toxic when expressed in other cell types.

The routes of administration and dosages described are intended only as a guide since a skilled practitioner will be able to determine readily the optimum route of administration and dosage for any particular patient and condition.

### E. Assay Methodologies

The expression cassettes of the invention can also be used in methods of scientific research. Thus, a further aspect of the present invention relates to methods of assaying gene function in eukaryotic cells, preferably mammalian cells, *either in vitro or in vivo.* The function of a heterologous could be determined by:
(a) introducing an expression cassette of the invention comprising said heterologous gene into a eukaryotic cell; and
(b) determining the effect of expression of said heterologous gene in said cell.

For example, the cell may have a temperature-sensitive defect in cell division. When an expression cassette comprising a heterologous gene according to the invention is introduced into the defective cell and the cell grown at the restrictive temperature, a skilled person will easily be able to determine whether the heterologous gene can complement the defect in cell division. Similarly, other known techniques can be applied to determine if expression of the heterologous gene can correct an observable mutant phenotype in the cell.

This procedure can also be used to carry out systematic mutagenesis of a heterologous gene to ascertain which regions of the protein encoded by the gene are involved in restoring the mutant phenotype.

This method can also be used in animals, for example mice, carrying so-called "gene knock-outs". A wild-type heterologous gene can be introduced into the animal using an expression cassette of the invention and the effect on the animal determined using various behavioural, histochemical or biochemical assays known in the art. Alternatively, a mutant heterologous gene can be introduced into either a wild-type or "gene knock-out" animal to determine if disease-associated pathology is induced. An example of this is the use of genes encoding prions to induce Creutzfeld-Jacob and other prion-type diseases in the central nervous system of rodents. Other disease models may include those for Alzheimer's disease, motor neurone disease or Parkinson's disease.

Since it is possible to introduce at least two different heterologous genes into a cell using one or more expression cassettes, it will also be possible to study the interaction between two or more gene products.

Thus, the methods of the present invention may be used in particular for the functional study of genes implicated in disease. One advantage of using the method of the invention to study gene function is that the ability to control the temporal expression of a particular gene means that it may be possible to ascertain at what stage in cell development or repair the gene needs to be expressed, if at all.

The invention will be described with reference to the following Examples which are intended to be illustrative only and not limiting.

### EXAMPLES

In Examples 1 to 8, the use of the LAT P2 region to give long-term expression is described for the following constructs:
1. An MMLV LTR promoter driving long term expression of lacZ from the LAT region in an ICP34.5 deleted, VMW65 trans-activating activity removed virus (virus strain 1764/pR14). Here, an MMLV LTR/LacZ cassette is inserted directly after LAT P2 in the LAT region.
2. A CMV promoter driving lacZ expression from the LAT region in an ICP27 deleted virus (virus strain 17+/D27/pR19lacZ). Here a CMV IE promoter lacZ cassette is inserted directly after LAT P2 in the LAT region.
3. A LAT P2/CMV promoter driving lacZ from the ICP27 locus, i.e. again with ICP27 deleted (virus strain 17+/D27/pR20). Here the LAT P2 region conferring long-term activity on a non-LAT promoter is used in isolation from other LAT sequences.
4. A LAT P2 region flanked downstream by an MMLV LTR promoter/GFP cassette and upstream by a LAT P1 promoter/lacZ cassette in the opposite orientation (the pR20.9 cassette), and inserted into the UL43 gene of a virus deleted for ICP34.5 and with an inactivating mutation in VMW65 (virus strain 1764/pR20.9).

### Material and Methods

### Viruses and cell lines

All viruses were prepared by standard methods (for example, see Coffin and Latchman, 1996; Coffin *et al.,* 1996) of homologous recombination followed by X-gal staining for lacZ activity, plaque purification, and Southern blotting to check correct genome structure of recombinant viruses.

### (a) Parent viruses and ICP27-complementing cell lines:

### 17+/D27w

An ICP27 deletion mutant not containing a marker gene was first produced by homologous recombination to remove the lacZ gene from a previously generated ICP27 deleted virus in which lacZ had been inserted into the ICP27 locus and selection of white plaques after staining with X-gal, all by standard methods. This virus was named 17+/D27w and is wild-type except with the deletion of nucleotides 113,273-117,854, removing the coding sequence for ICP27 (UL54) and genes UL55 and UL56, which are non-essential, and which must therefore be grown on an ICP27-expressing cell-line. Nucleotide numbers refer to the HSV1 strain 17+ sequence (Genbank no. HE1CG).

ICP27 deleted viruses were generated and stocks prepared by growth on the ICP27 complementing BHK cell line B130/2 previously generated by co-transfection of plasmid pSG130BS (Sekulovich *et al.,* 1988) DNA with neomycin resistance encoding plasmid pMamNeo (Invitrogen) into BHK cells and the selection of neomycin resistant clones. A clone highly permissive for the growth of an HSV1 ICP27 deletion mutant (B130/2) was selected for virus growth. pSG130BS carries a *Bam*HI/*Sac*I fragment from HSV1 (nucleotides 113,322-115,743) encoding the complete ICP27 coding sequence and part of UL55.

### 1764

HSV strain 1764 has been previously described (Coffin *et al.,* 1996). It is deleted for the gene encoding ICP34.5 (MacLean *et al.,* 1991) and has an inactivating mutation in the gene encoding VMW65 (Ace *et al.,* 1989), the virion transactivating protein. ICP34.5 is non-essential for virus growth in vitro and the VMW65 can be complemented by inclusion of hexamethylene-bisacetamide in the media (McFarlane *et al.,* 1992) and so can be grown on BHK cells.

### Example 1 - Construction of 1764/pR14

Virus strain 1764/pR14 was produced by co-transfection of purified 1764 genomic DNA with plasmid pR14 into BHK cells and selection of blue plaques after X-gal staining. pR14 was produced by insertion of MMLV LTR/lacZ sequences into pNot 3.5. pNot 3.5 contains a 3.5 Kb *Not*I fragment from the LAT region of HSV1 (nucleotides 118439-122025) cloned into the *Not*I site of pGem5 (Promega). The MMLV LTR/lacZ insertion was made in two stages:
1: The lacZ gene (*Hind*III*-Bam*HI) from pCH110 (Pharmacia) was inserted into the *Hind*III site of pJ4 (containing MMLV LTR promoter/polylinker/SV40 polyA sequences; (Morgenstern and Land, 1990)) giving pJ4lacZb.
2: The MMLV LTR/lacZpolyA from pJ4lacZ was inserted into pNot 3.5 at the *Bbs*I site (after LAT P2) by excision from pJ4lacZ with *Nhe*I and *Pst*I, giving pR14.

### Orientation: LAT P1/LAT P2/LTR/lacZ.

### Example 2 - Construction of 17+/D27/pR19lacZ

Virus strain 17+/D27/pR19lacZ was produced by co-transfection of purified 17+/D27w genomic DNA with plasmid pR19lacZ into B130/2 cells and selection of blue plaques after X-gal staining. pR19lacZ was produced by insertion of a CMV IE promoter/lacZ/polyA cassette into the *Bst*XI site of pNot 3.5, i.e. after LAT P2. First the lacZ gene (*Hin*dIII - *Bam*HI) from pCH110 (Pharmacia) was cloned into pcDNA3 (Invitrogen, containing CMV IE promoter/polylinker/polyA sequences) between the *Bam*HI and *Hin*dIII sites. The CMV IE promoter/lacZ/polyA cassette was then excised with *Nru*I and *Bbs*I and inserted into pNot 3.5 at the *Bst*XI site. Orientation: LAT P1/LAT P2/CMV/lacZ/polyA.

### Example 3 - Construction of 17+/D27/pR20

Virus strain 17+/D27/pR20 was produced by co-transfection of purified 17+/D27w genomic DNA with plasmid pR20 into B 130/2 cells and selection of blue plaques after X-gal staining. pR20 was constructed by insertion of a LAT P2/CMV IE promoter/LacZ/poly A cassette *(Pst*I *- Srf*I from pR19lacZ. The *Srf*I site is just after the *BstX*I site in pNot 3.5.) into pDMN. pDMN was produced by deleting a *Not*I/*Xmn*I fragment from the *EcoR*1 B fragment of the HSV1 genome cloned into pACYC184 (NBL), to leave a fragment which includes the gene for ICP27 and flanking sequences (HSV1 strain 17+ nucleotides 11095-118439). A pair of *Mlu*I fragments encoding the entire ICP27 coding sequence together with the non-essential genes UL55 and 56 (nucleotides 113273-116869) were then removed by digestion with *Mlu*I and religation. The LAT P2/CMV IE promoter/LacZ/poly A cassette was then inserted at the *Mlu*I site.

### Example 4 - Construction of 1764/pR20.9

Virus strain 1764-pR20.9 was produced by co-transfection of purified 1764 genomic DNA with plasmid pR20.9/43, resulting in insertion of the pR20.9 cassette into the UL43 gene of virus strain 1764. This construct comprises two heterologous genes, The first gene, green fluorescent protein (GFP) is under control of the MMLV LTR promoter whilst the second gene, lacZ, is under control of the LAT P1 promoter. These flank the LAT P2 region in opposite orientations such that the respective genes (lacZ and GFP) are transcribed in opposite directions away from the central LAT P2 region.

### Plasmid pR20.9/43 was constructed by:

(i) Insertion of GFP into plasmid pcDNA3 (Invitrogen) by excision of the GFP gene from plasmid pEGFP-N1 (Clontech) with *Age*I and *Not*I and inserting between the *EcoR*1 and *Not*I sites of pcDNA 3, giving plasmid pcDNA3GFP. The start of the GFP gene is orientated next to the CMV promoter.
(ii) Insertion of the MMLV promoter from pJ4 (*Nhe*I*-Hind*III) into the *BamH*I site of pcDNA3GFP, giving pcMMLVGFP, orientated such that the *Nhe*I site from pJ4 is next to the CMV promoter.
(iii) insertion of the MMLV/GFP/pA cassette from pcMMLVGFP into pNot3.5 by excision with *Hind*III and *Bbs*I and insertion between the *BstX*I sites of pNot3.5, i.e. after the LAT P2 sequence, orientated such that the MMLV promoter is next to the LAT P2 region, giving plasmid p3.5MG.
(iv) insertion of the LAT P1 promoter (nt 118,179-118,877) from Ddel-StyI between the *EcoRV* and *Spe*I sites of pGem5 (Promega) - orientation *Sty*I ligated to *EcoRV, Dde*I ligated to *Spe*I giving pGem5PI.
(v) insertion of lacZ *(Hind*III*-Bam*HI*)* from pCH110 (Pharmacia) into the *Nco*I site of pGEMSPI - orientation *Hind*III next to the LAT P1 sequence - giving pP1/lacZ.
(vi) insertion of an oligonucleotide encoding an *Srf*I site (5' - GCCCGGGCCATG) into the *Sph*I site of pP1/lacZ, giving pP1/lacZSrf.
(vii) insertion of a LAT P2/MMLV/GFP/pA cassette *(Pst*I fragment) from p3.5MG into the *Nsi*I site of pP1/lacZSrf, orientated such that the LAT P2 region from p3.5MG is next to the LAT P1 region from pP1/lacZSrf, giving plasmid pR20.9.
(viii) insertion of the pR20.9 cassette into UL43 flanking regions by excision with *Srf*I and insertion into the unique *Nsi*I site of p35, giving pR20.9/43. p35 contains HSV1 nts 91,610-96,751 (BamHI-EcoRI) cloned into pGem1 (Promega).

### Example 5 - Inoculation of mice/rats with 1764/pR14/TH

### Peripheral nervous system.

1764/pR14 was tested by footpad inoculation of mice (1x10⁷ pfu) followed by subsequent sacrifice and dissection of lumbar dorsal root ganglia. After fixing and X-gal staining by standard methods (Coffin *et al.,* 1996) blue staining could be seen after 2 days, 2 weeks, 1 month and 2 months (longest time tested) in lumbar ganglia L4 and L5, indicating long-term promoter activity.

### Central nervous system

After stereotaxic inoculation (5x10⁵ pfu in 2 µl) to the rat striatum (200-220 g Lewis rats) 1764/pR14 gave blue staining following fixing and X-gal staining at 2 days, 2 weeks, 1 month and 2 months (longest time tested). This again indicated long-term promoter activity.

### Example 6 - Inoculation of rats with 17+/D27/pR19

### Central nervous system

After stereotaxic inoculation (5x10⁶ pfu in 2 µl) to the rat striatum (200 to 220 g Lewis rats) 17+/D27/pR19 gave strong blue staining after fixing and X-gal staining at 2 days, 2 weeks, 1 month and 2 months (longest time tested). This showed that the CMV IE promoter, when placed after LAT P2, like the MMLV LTR could also give long term activity.

### Example 7 - Inoculation of rats with 17+/D27/pR20

### Central nervous system

After stereotaxic inoculation (5x10⁶ pfu in 2 µl) to the rat striatum (200-220 g Lewis rats) 17+/D27/pR20 also gave strong blue staining after fixing and X-gal staining at 2 days, 2 weeks and 1 month (longest time tested). Importantly, these indicate that LAT P2 can give long term activity from a proximal promoter when inserted elsewhere in the herpes genome, ie not in the LAT region.

### Example 8 - inoculation of mice with 1764/pR20.9

### Peripheral nervous system

1764/pR20.9 was tested by footpad inoculation of mice (1x10⁷ pfu) followed by subsequent sacrifice and dissection of lumbar dorsal root ganglia. After fixing strong green fluorescence could be seen under fluorescence microscopy (fluoroscein optics) and after X-gal staining blue staining could be seen in the same cells after 2 days, 2 weeks, 1 month and 2 months (longest time tested) in lumbar ganglia L4 and L5, and to a lesser extent in other DRGs. This indicated that both promoters were active in the long term and showed that pairs of genes could be efficiently expressed using a cassette of the invention.

### References

1. Coffin RS and Latchman DS. Herpes simplex virus-based vectors. In: Latchman DS (ed). Genetic manipulation of the nervous system. Academic Press: London, 1996, pp 99-114.
2. MacLean AR *et al.* Herpes simplex virus type I deletion variants 1714 and 1716 pinpoint neurovirulence related sequences in Glasgow strain 17+ between immediate early gene I and the 'a' sequence. J Gen Virol 1991; 72: 632-639.
3. Morgenstern JP and Land H. A series of mammalian expression vectors and characterisation of their expression of a reporter gene in stably and transiently transfected cells. NAR 1990.; 18: 1068.
4. Sekulovich RE *et al.,* 1988, J. Virol. 64: 3916-3926.
5. Ace C *et al.* Construction and characterisation of a herpes simplex virus type I mutant unable to transinduce immediate early gene expression. J Virol 1989; 63: 2260-2269.
6. McFarlane M, Daksis JI, Preston CM. Hexamethylene bisacetamide stimulates herpes-simplex virus immediate early gene-expression in the absence of trans-induction by VMW65. J Gen Virol 1992; 73: 285-292.
7. Rice, SA and Knipe DM., 1990, J. Virol 64: 1704-1715.
8. Chou, J., Poon, APW, Johnson, J. and Roizman B. Differential response of human cells to deletions and stop codons in the γ₁34.5 gene of herpes simplex virus. J. Virol. 1994; 68: 8304-8311.
9. Smith, IL *et al.,* 1992, Virology 186: 74-86.
10. Coffin RS *et al.,* 1996, Gene Therapy 3: 886-891.
11. Chou, J. and Roizman, B. The γ₁34.5 gene of herpes simplex virus 1 precludes neuroblastoma cells from triggering total shutoff of protein synthesis characteristic of programmed cell death in neuronal cells. PNAS 1992; 89: 3266-3270.
12. Gossen M and Bujard H, 1992, PNAS 89: 5547-5551.
13. Gossen M *et al.,* 1995, Science 268: 1766-1769.
14. Sambrook et *al.,* 1989, Molecular Cloning - A Laboratory Manual. Cold Spring Harbor Press.
15. Goins, W. F. *et al.,* 1994, J. Virol. 68: 2239-2252.

## Claims

1. An expression cassette comprising:
- a herpes simplex virus (HSV) latency-associated transcript (LAT) P2 region consisting of HSV type 1 nucleotides 118,866-120,219 or of a fragment or derivative thereof which is capable of providing for long-term expression of a heterologous gene in the cassette;
- a first promoter; and
- a first heterologous gene
operably linked in that order for use in a method of treating the human or animal body.

2. An expression cassette according to claim 1 further comprising a second promoter and a second heterologous gene operably linked in that order to said HSV LAT P2 region and in the opposite orientation to the first promoter and first heterologous gene wherein said second promoter is the same as or different to the first promoter and said second heterologous gene is the same as or different to said first heterologous gene.

3. An expression cassette according to claim 2 wherein the product of said first heterologous gene regulates the expression of said second heterologous gene under suitable physiological conditions.

4. An expression cassette according to any one of the preceding claims wherein said first and/or second promoter is a non-LAT promoter.

5. An expression cassette according to any one of the preceding claims wherein said first and/or second promoter is a viral promoter or a mammalian promoter permitting expression of said heterologous gene in a mammalian cell.

6. An expression cassette according to claim 5 wherein said mammalian promoter is a tissue-specific promoter.

7. An expression cassette according to claim 5 or 6 wherein said mammalian cell is a cell of the central or peripheral nervous system, eye, heart or skeletal muscle of a mammal.

8. An expression cassette according to any one of the preceding claims wherein said first and/or second heterologous gene encodes a polypeptide of therapeutic use.

9. An expression cassette according to any one of the preceding claims wherein said heterologous gene is selected from genes encoding polypeptides which are cytotoxic, polypeptides which are capable of converting a precursor prodrug into a cytotoxic compound, proteins involved in the regulation of cell division, enzymes involved in cellular metabolic pathways, transcription factors and heat shock proteins.

10. An expression cassette according to any one of the preceding claims wherein said LAT P2 region is a homologous region of HSV type 2.

11. A nucleic acid vector comprising an expression cassette as defined in any one of the preceding claims for use in a method of treating the human or animal body.

12. A vector according to claim 11 further comprising mammalian or HSV genomic sequences flanking said expression cassette.

13. A herpes virus strain comprising an expression cassette as defined in any one of claims 1 to 10 for use in a method of treating the human or animal body.

14. A herpes virus strain according to claim 13 wherein said viral strain is an HSV strain.

15. A herpes virus strain according to claim 13 or 14 wherein said LAT P2 region is the endogenous HSV LAT P2 region.

16. Use of an expression cassette as defined in any one of claims 1 to 10, a vector as defined in claim 11 or 12 or a herpes virus strain according to any one of claims 13 to 15 in the treatment of a disorder of, or injury to, the nervous system including Parkinson's disease, spinal injury or stroke, or a disease of the eye, heart or skeletal muscle, or a malignancy.

17. A pharmaceutical composition comprising an expression cassette as defined in any one of claims 1 to 10, a vector as defined in claim 11 or 12 or a herpes virus strain as defined in any one of claims 13 to 15 together with a pharmaceutically acceptable carrier or diluent.

18. An expression cassette according to any one of claims 1 to 7 wherein said first and/or second heterologous gene encodes a polypeptide comprising at least one epitope.

19. An expression cassette according to claim 18 wherein said polypeptide is derived from a pathogenic organism.

20. A vector comprising an expression cassette as defined in claim 18 or 19 for use in a method of treating the human or animal body.

21. A herpes virus strain comprising an expression cassette as defined in claim 18 or 19 for use in a method of treating the human or animal body.

22. The use of an expression cassette as defined in claim 18 or 19, a vector as defined claim 20 or herpes virus strain as defined in claim 21 in the manufacture of a medicament for use in a method of vaccinating a mammal.

23. A vaccine comprising an expression cassette as defined in claim 18 or 19, a vector as defined in claim 20 or a herpes virus strain as defined in claim 21 together with a pharmaceutically acceptable carrier or diluent.
